# EUROPÄISCHE PATENTSCHRIFT

(11) **EP 2 301 622 B1**
(45) Veröffentlichungstag und Bekanntmachung des Hinweises auf die Patenterteilung: **09.03.2016**
(21) Anmeldenummer: 10181328.5
(22) Anmeldetag: 15.10.2004
(51) Int. Cl.: A61J 1/10, A61M 39/20, A61M 39/26, A61M 39/04

(54) **Konnektor für medizinische Flüssigkeiten enthaltende Verpackungen und Verpackung für medizinische Flüssigkeiten**
Connector for packaging containing medical fluids and packaging for medical fluids
Connecteur pour des emballages contenant des fluides médicaux et emballage pour fluides médicaux

(30) Priorität: 15.10.2003 DE 10348016
(43) Veröffentlichungstag der Anmeldung: 30.03.2011
(62) Teilanmeldung aus: 04790456.0
(73) Patentinhaber: Fresenius Kabi Deutschland GmbH, 61352 Bad Homburg (DE)
(72) Erfinder: Brandenburger, Torsten, 61203 Reichelsheim (DE); Rahimy, Ismael, 61169 Friedberg (DE)
(74) Vertreter: Fresenius Kabi Deutschland GmbH

(56) Entgegenhaltungen:
- WO-A-96/23545
- WO-A-2004/084793
- US-A- 5 088 995
- US-A- 5 100 394
- US-A- 6 142 446
- US-B1- 6 183 448
- US-B1- 6 325 782

## Beschreibung

Die Erfindung bezieht sich auf einen Konnektor für medizinische Flüssigkeiten enthaltende Verpackungen, insbesondere Infusions- oder Transfusionsbeutel, der zum Zuspritzen einer Flüssigkeit in den Beutel dient. Darüber hinaus betrifft die Erfindung eine Verpackung für medizinische Flüssigkeiten, insbesondere einen Infusions- oder Transfusionsbeutel, mit einem derartigen Zuspritzteil.

Ein Infusionsbeutel mit einem Zuspritzteil und einem Entnahmeteil ist aus der WO 96/23545 bekannt. Der Zuspritzteil dient zum Zuführen eines Medikamentes mittels einer Injektionsspritze, die über eine dünne Kanüle (Nadel) verfügt. Er umfasst einen rohrförmigen Anschlussteil, der von einer als Abbrechteil ausgebildeten Schutzkappe verschlossen ist. Im Öffnungsbereich des Anschlussteils sitzt ein selbstabdichtendes Septum. Eine durchstechbare Membran in dem Anschlussteil verhindert, dass das Septum vor dem Gebrauch des Infusionsbeutel mit der Lösung in Berührung kommt. Der Entnahmeteil, der zur Entnahme der Lösung mittels eines Spike dient, weist ein selbstabdichtendes Septum nicht auf.

Die bekannten Zuspritzteile zeichnen sich dadurch aus, dass das selbstabdichtende Septum im Öffnungsbereich des rohrförmigen Anschlussteils derart angeordnet ist, dass es mit dem Anschlussteil im wesentlichen bündig abschließt. Nach dem Abbrechen des Abbrechteils liegt das Septum frei. Zum Zuspritzen eines Medikamentes wird das Septum von einer Injektionsnadel durchstochen.

Derartige Zuspritzteile sind beispielsweise auch aus der DE 197 28 775 A1 oder WO 96/23545 oder WO 2009/084793 oder DE 100 30 474 C1 bekannt.

Die bekannten Zuspritzteile haben sich in der Praxis bewährt. Aus der Verwendung einer Injektionsnadel zum Zuspritzen eines Wirkstoffes ergeben sich aber Nachteile. Zum einen besteht die Gefahr, dass sich die Verbindung zwischen Injektionsnadel und Septum infolge eines unbeabsichtigten Zugs an der Spritze oder eines Überdrucks im Beutelinneren löst. Zum anderen besteht eine erhöhte Verletzungsgefahr für das Pflegepersonal durch die Injektionsnadel. Auch die Verpackung kann bei unsachgemäßer Handhabung durch die Nadel beschädigt werden. Das Zuführen eines zähflüssigen Wirkstoffes ist darüber hinaus auf Grund des kleinen Querschnitts der Injektionsnadel erschwert. Infolge des kleinen Querschnitts erfordert das Zuführen eines dünnflüssigen Wirkstoffes relativ viel Zeit.

Zum Anschluss medizinischer Geräte sind in der Medizintechnik Kegelverbindungen mit einem Kegelschaft und einer Kegelhülse bekannt, deren Kegelflächen normiert sind. Die nicht verriegelbaren Kegelverbindungen mit normierten Kegelflächen werden als Luer-Konnektoren und die verriegelbaren Kegelverbindungen als Luer-Lock-Konnektoren bezeichnet. Die Luer- oder Luer-Lock-Konnektoren mit Kegelschaft werden als männliche Konnektoren und die Konnektoren mit Kegelhülse als weibliche Konnektoren bezeichnet.

Die DE 196 36 610 A1 beschreibt eine Einfüllvorrichtung für ein Zufuhrsystem von Wirkstoffen, das über eine Leitung mit einem Systemreservoir verbunden wird. Der Wirkstoff wird mittels einer konventionellen Spritze ohne Injektionsnadel der Einfüllvorrichtung zugeführt. Der Anschluss der Spritze an die Einfüllvorrichtung erfolgt mit einer Luer-Lock-Kegelverbindung.

Aus der WO 00/02517 ist ein steriler Konnektor zum Anschluss des Spike eines Überleitgeräts bekannt, der über einen Basisteil mit einer kanalförmigen Ausnehmung verfügt, die von einem Abbrechteil verschlossen wird. Der Basisteil weist ein Aufnahmestück für den Spike auf, das im wesentlichen zylindrisch ausgebildet ist. Der bekannte Konnektor ist weder für den Anschluss einer Luer-Lock-Spritze bestimmt noch geeignet.

Die US 6 186 997 beschreibt einen Infusionsbeutel mit einem Schlauchstück, das am Ende mit einer geschlitzten Membran verschlossen ist. Mit dem Endstück des Schlauchstücks ist eine Verschlusskappe verschraubt.

Der Erfindung liegt die Aufgabe zugrunde, einen kostengünstig herzustellenden und einfach und sicher zu handhabenden Konnektor für medizinische Flüssigkeiten enthaltende Verpackungen, insbesondere Infusions- oder Transfusionsbeutel zu schaffen, der ein schnelles Zuführen, insbesondere eines zähflüssigen Wirkstoffes erlaubt und mit dem die Verletzungsgefahr sowohl für das Pflegepersonal als auch die Verpackung gering ist.

Eine weitere Aufgabe der Erfindung liegt darin, eine einfach und sicher zu handhabende Verpackung für medizinische Flüssigkeiten, insbesondere Infusions- oder Transfusionsbeutel, zu schaffen, in die sich auch zähflüssige Wirkstoffe ohne die Gefahr einer Verletzung des Pflegepersonals oder der Verpackung schnell zu spritzen lassen.

Die Lösung der obigen Aufgaben erfolgt erfindungsgemäß mit den Merkmalen des Patentanspruchs 1 bzw. 15. Vorteilhafte Ausführungsformen der Erfindung sind Gegenstand der Unteransprüche.

Der erfindungsgemäße Konnektor erlaubt den Anschluss einer konventionellen Spritze mit einer Kegelverbindung nach Art einer Luer-Kegelverbindung oder einer Luer-Lock-Kegelverbindung, die nicht über eine Injektionsnadel verfügt. Da eine Injektionsnadel mit kleinem Querschnitt nicht erforderlich ist, können auch Wirkstoffe größerer Viskosität schnell zugespritzt werden. Die Gefahr der Verletzung für Pflegepersonal und Verpackung besteht nicht. Die Möglichkeit der Verschraubung von Spritze und Konnektor stellt sicher, dass sich die Verbindung nicht löst. Der Konnektor verfügt über eine selbstabdichtende, geschlitzte Membran, die unterhalb der oberen, anschlussseitigen Öffnung angeordnet ist. Oberhalb der selbstabdichtenden Membran ist der Anschlussteil des Konnektors als ein Anschlussstück mit einem Innenkonus für den Kegelschaft der Spritze ausgebildet. Die selbstabdichtende Membran ist zur abdichtenden Aufnahme des Kegelschaftes der Spritze durchgehend geschlitzt. Nach dem Herausziehen des Kegelschaftes schließt sich die Membran wieder und verhindert somit ein Auslaufen der Flüssigkeit aus der Verpackung.

Eine vorteilhafte Ausführungsform der Erfindung sieht vor, dass der Abbrechteil über eine Ringbruchzone an den Anschlussteil angeschlossen ist, so dass der Abbrechteil zwar einen sicheren Halt hat, sich aber dennoch relativ leicht lösen lässt.

Bei einer besonders bevorzugten Ausführungsform setzt sich der Anschlussteil aus einem unteren Teilstück und einem oberen Teilstück zusammen, wobei die Teilstücke einschnappend festgelegt sind. Vorzugsweise ist die selbstabdichtende Membran zwischen dem unteren und oberen Teilstück klemmend gehalten. Dadurch wird die Montage vereinfacht. Der Anschlussteil kann aber auch einstückig sein.

Die selbstabdichtende Membran weist vorzugsweise einen unteren ringförmigen Abschnitt und einen obereren tellerförmigern Abschnitt auf. Vorteilhafterweise ist der ringförmige Abschnitt der Membran zwischen dem unteren und oberen Teilstück des Anschlussteils eingespannt. Damit hat die Membran einen sicheren Halt. Der obere tellerförmige Abschnitt der Membran weist vorzugsweise eine muldenförmige Vertiefung auf. Die muldenförmige Vertiefung stellt zum einen sicher, dass der Kegelschaft der Spritze sicher geführt wird und gewährleistet zum anderen, dass die Membran nach dem Herausziehen des Kegelschaftes sicher abdichtet. Es hat sich in Versuchen gezeigt, dass die besondere Ausbildung der Membran für den sofortigen Wiederverschluss ausschlaggebend ist, wobei mit zunehmendem Innendruck in der Verpackung die Abdichtung der Membran noch erhöht wird.

Vorzugsweise schließt sich an den oberen tellerförmigen Abschnitt der selbstabdichtenden Membran ein mittleres Zwischenstück an, das in den unteren ringförmigen Abschnitt der Membran übergeht. Damit sind der obere und untere Abschnitt der Membran elastisch miteinander verbunden, so dass sich die Membran beim Einführen sich Kegelschaftes der Spritze derart verformt, dass sie gegenüber dem Anschlussteil sicher abdichtet.

Der Innendurchmesser des ringförmigen Abschnitts der selbstabdichtenden Membran ist vorzugsweise kleiner als der Innendurchmesser der kanalförmigen Ausnehmung des Anschlussteils. Es hat sich gezeigt, dass dadurch der Wiederverschluss der Membran weiter verbessert wird.

Der Innenkonus des Anschlussstücks und die selbstabdichtende Membran des Anschlussteils sind vorzugsweise weiterhin derart ausgebildet und angeordnet, dass der in den Innenkonus eingesetzte Kegelschaft der Spritze die geschlitzte Membran zwar öffnet, aber nicht durchdringt.

Der Abbrechteil des Konnektors ist erfindungsgemäß als flaches Griffstück ausgebildet, um mit Daumen und Zeigefinger gehalten werden zu können. Dadurch wird die Handhabung vereinfacht.

Der Konnektor ist zweckmäßigerweise ein Spritzgießteil, das in großen Stückzahlen kostengünstig hergestellt werden kann.

Im Folgenden wird ein Ausführungsbeispiel der Erfindung unter Bezugnahme auf die Zeichnungen näher erläutert:
Es zeigen:

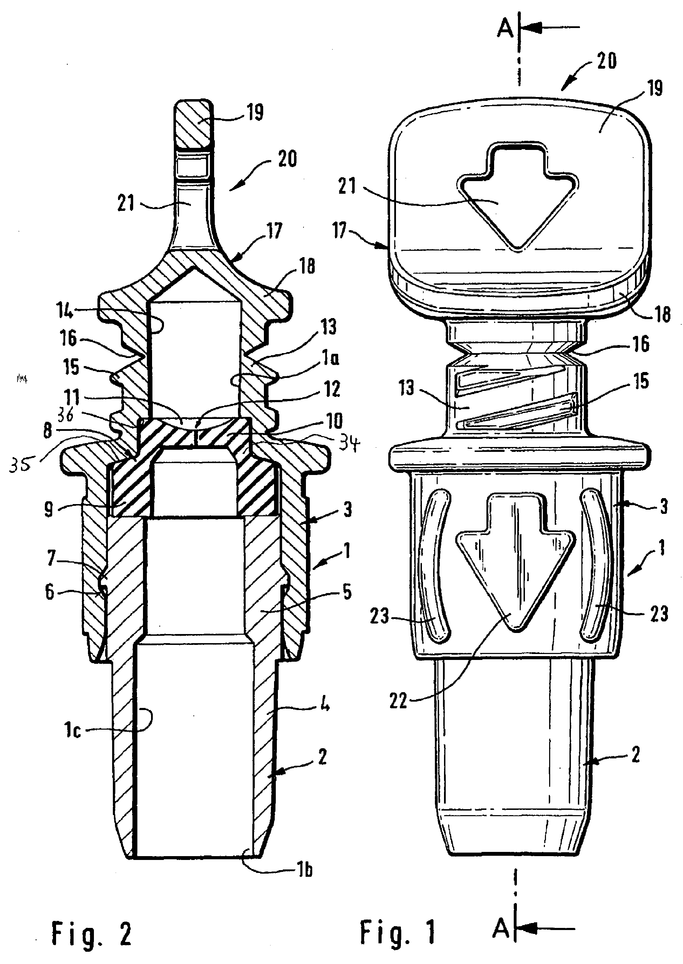

   - Figur 1: Ein als Zuspritzteil ausgebildeter Konnektor für medizinische Flüssigkeiten enthaltende Verpackungen in der Seitenansicht,
   - Figur 2: einen Schnitt durch den Konnektor von Figur 1 entlang der Linie A-A,
   - Figur 3: einen Infusionsbeutel mit dem Konnektor von Figur 1,
   - Figur 4a: eine Luer-Spritze und einen Konnektor mit abgebrochenem Abbrechteil,
   - Figur 4b: den Ausschnitt A von Figur 4a in vergrößerter Darstellung,
   - Figur 5a: die Luer-Spritze und den Konnektor von Figur 4a beim Einführen des Kegelschaftes der Spritze in den Konnektor,
   - Figur 5b: den Ausschnitt A von Figur 5a in vergrößerter Darstellung,
   - Figur 6a: die Luer-Spritze und den Konnektor von Figur 4a, wobei der Kegelschaft der Spritze in den Konnektor eingesetzt ist und
   - Figur 6b: den Ausschnitt A von Figur 6a in vergrößerter Darstellung.

Der erfindungsgemäße Konnektor ist als Zuspritzteil zum Zuspritzen eines Wirkstoffes in eine Verpackung ausgebildet, die eine medizinische Flüssigkeit enthält, insbesondere ein Infusions- oder Transfusionsbeutel. Der Konnektor 20 weist einen Anschlussteil 1 mit einer kanalförmigen Ausnehmung 1c auf, der in der Gebrauchslage aus einem verpackungsseitigen unteren Teilstück 2 und einem anschlussseitigen oberen Teilstück 3 zusammengesetzt ist. Der Anschlussteil 1 weist folglich eine obere und untere Öffnung 1a und 1b auf. Der Konnektor ist ein Spritzgießteil aus Polypropylen.

Das untere Teilstück 2 des Anschlussteils 1 weist einen unteren und oberen zylindrischen Abschnitt 4, 5 auf, wobei der untere Abschnitt einen etwas größeren Innendurchmesser als der obere Abschnitt, aber einen etwas kleineren Außendurchmesser als der obere Abschnitt hat. Der untere Abschnitt 4 kann in einen Anschlussstutzen eines Folienbeutels eingeschoben und mit dem Stutzen verschweißt oder verklebt werden. Er kann aber auch direkt in den Folienbeutel ohne Stutzen eingeschweißt oder eingeklebt werden.

Das obere Teilstück 3 des Anschlussteil 1 ist einschnappend auf dem unteren Teilstück 2 festgelegt. Hierzu weist die Innenwand des oberen Teilstück eine umlaufende Nut 6 auf, in die beim Zusammenpressen der beiden Teilstücke ein umlaufender Vorsprung 7 an der Außenwand des oberen Teilstücks 3 einschnappt.

Zwischen dem unteren und oberen Teilstück 2, 3 ist eine selbstabdichtende Membran 8 aus einem elastischen Material, die auch als Septum bezeichnet wird, vorzugsweise unter leichter elastischer Verformung derselben klemmend gehalten. Die Membran 8 weist einen ringförmigen unteren Abschnitt 9 auf, der zwischen dem unteren und oberen Teilstück 2,3 des Anschlussteils 1 eingespannt ist. An dem unteren ringförmigen Abschnitt 9 der Membran 8 schließt sich ein mittleres Zwischenstück 34 an, das in einen oberen tellerförmigen Abschnitt 10 übergeht, der eine muldenförmige Vertiefung 11 hat.

Der Anschlussteil 1 weist einen unteren und einen oberen nach innen vorspringenden Ansatz 35, 36 auf. An dem unteren Ansatz 35 stützt sich der ringförmige Abschnitt 9 und an dem oberen Ansatz 36 der tellerförmige Abschnitt 10 der Membran 8 ab. Vorzugsweise sind beide Abschnitte 9, 10 der Membran 8 gegen die Ansätze 35, 36 des Anschlussteils 1 federnd vorgespannt. Der Innendurchmesser des ringförmigen Abschnitts 9 der Membran 8 ist kleiner als der Innendurchmesser der kanalförmigen Ausnehmung 1c des Anschlussteils 1.

Im Zentrum des tellerförmigen Abschnitts ist die Membran mit einem durchgehenden Schlitz 12 versehen. Die Membran 10 kann mit nur einem querverlaufenden Schlitz versehen oder auch kreuzweise oder sternförmig geschlitzt sein. Der Schlitz erstreckt sich vorzugsweise nahezu über den gesamten Querschnitt des tellerförmigen Abschnitts. Die muldenförmige Vertiefung 11 in dem tellerförmigen Abschnitt 10 kann eine gebogene oder geradlinige Flanke haben.

Oberhalb der Membran 10 ist der Anschlussteil 1 als ein Anschlussstück 13 ausgebildet, das eine Innenkonus 14 und vorzugsweise ein Außengewinde 15 aufweist. Innenkonus und Außengewinde entsprechen dem Kegelschaft der Luer-Lock-Kegelverbindung einer konventionellen Spritze, so dass der Kegelschaft der Luer-Lock-Spritze dichtend in den Innenkonus des Anschlussstücks eingeschoben und mit dem Anschlussteil sicher verschraubt werden kann. Für den Anschluss einer Luer-Spritze, die nicht über eine Schraubverbindung verfügt, kann das Außengewinde auch entfallen.

Über eine Ringbruchzone 16 schließt sich an das Anschlussstück 13 ein kappenförmiger Abbrechteil 17 an, das die obere Öffnung 1a des Anschlussstücks verschließt. Der Abbrechteil bildet einen Originalitätsverschluss für den Konnektor. Der Abbrechteil 17 weist ein unteres rotationssymmetrisches Basisteil 18 und ein oberes flaches Griffstück 19 auf. Das flache Griffstück 19 ist mit einer Aussparung 21 versehen, die nach Art eines nach unten zeigenden Pfeils ausgebildet ist, der den Konnektor als Zuspritzteil kennzeichnet. Auch das obere Teilstück 2 des Anschlussteils weist zur Kennzeichnung der Flussrichtung eine erhabene Struktur 22 auf, die nach Art eines nach unten zeigenden Pfeils ausgebildet ist. Der Pfeil 22 ist zwischen zwar vorspringenden Stegen 23 angeordnet, die eine Griffmulde bilden.

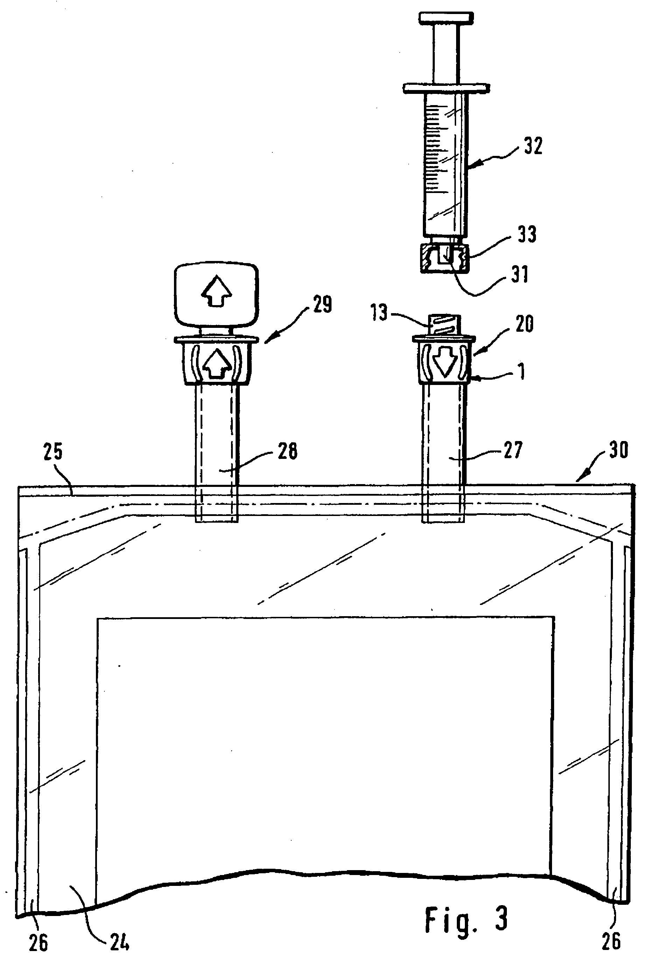

Figur 3 zeigt einen Infusionsbeutel 30 zusammen mit dem Zuspritzteil 20. Der Infusionsbeutel 30 besteht aus zwei Folienlagen 24, die am unteren und oberen Rand 25 sowie den längslaufenden Rändern 26 miteinander verschweißt sind. In den oberen Rand 25 des Infusionsbeutels ist ein Anschlussstutzen 27 für den Zuspritzteil 20 und ein Anschlussstutzen 28 für ein Entnahmeteil 29 eingeschweißt. Der rohrförmige Anschlussteil 1 des Zuspritzteils 20 ist in den Anschlussstutzen 27 eingeschoben und mit dem Stutzen beim Sterilisieren verschweißt. Das rohrförmige Anschlussteil des Zuspritzteils kann aber auch an ein nach Art eines Schiffchens ausgebildetes oder rundes Einsatzstück angeformt sein, das zwischen den beiden Folienlagen eingeschweißt ist.

Zum Zuspritzen einer Wirksubstanz in die Infusionslösung wird der Abbrechteil 17 des Zuspritzteils 20 durch Drehen oder Brechen desselben abgedreht bzw. abgebrochen, so dass die selbstabdichtende Membran 8 freiliegt. In den Innenkonus 14 des Anschlussstücks 13 wird der Kegelschaft 31 einer konventionellen Luer-Lock-Spritze 32 eingeschoben, wobei die selbstabdichtende Membran durchstoßen wird. Dabei dichtet der Kegelschaft der Spritze gegenüber dem Innenkonus 14 des Anschlussstücks 13 ab. Anschließend wird die Schraubkappe 33 der Spritze 5 auf das Außengewinde 5 des Anschlussstücks 17 aufgeschraubt, so dass die Spritze an dem Zuspritzteil 20 befestigt ist. Daraufhin kann mittels der Spritze eine Wirksubstanz zugespritzt und die Spritze wieder abgenommen werden, wobei die Membran das Zuspritzteil dicht verschließt.

Neben dem Zuspritzteil weist der Infusionsbeutel noch einen Entnahmeteil 29 zum Entnehmen der Infusionslösung auf, der mit dem Stutzen 28 verschweißt ist. Der Entnahmeteil ist aber nicht Gegenstand der Erfindung.
Im Folgenden wird die Funktionsweise der selbstabdichtenden Membran im Einzelnen beschrieben.

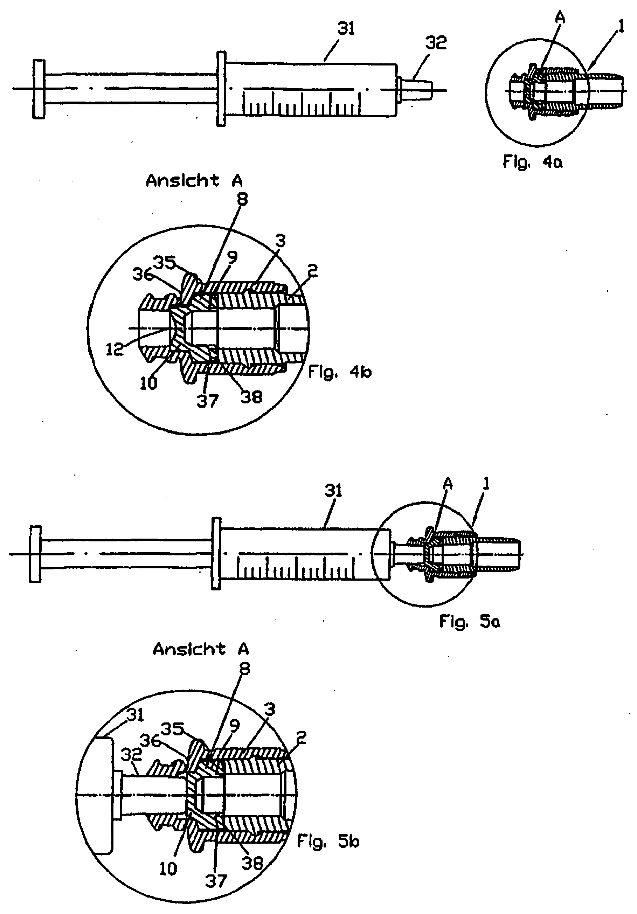

Vor dem Einführen des Kegelschaftes 31 einer Luer-Spritze 32 stützen sich der ringförmige und tellerförmige Abschnitt 9, 10 der Membran 8 an den Ansätzen 35, 36 des Anschlussteils 1 ab (Fig. 4a und 4b). Beim Einführen des Kegelschaftes 31 der Spritze 32 wird der tellerförmige Abschnitt 10 der Membran 8 unter Verformung desselben zurückgedrückt (Fig. 5a und 5b). Dabei verliert zuerst der tellerförmige Abschnitt 10 und dann der ringförmige Abschnitt 9 der Membran 8 den Kontakt mit dem Ansatz 35 bzw. 36. Dabei wird der tellerförmige Abschnitt der Membran gegen die Wandung des Anschlussteils gedrückt, so dass die Öffnung in dem Anschlussteil sicher abgedichtet wird.

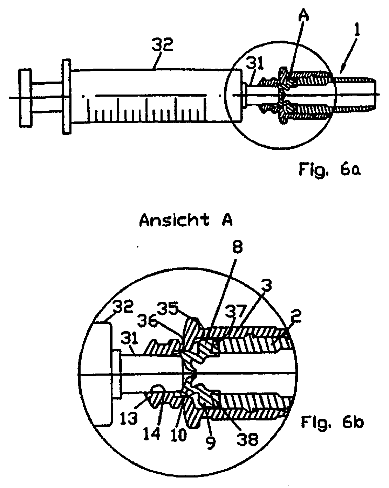

Der Innenkonus 14 des Anschlusstücks 13 und die selbstabdichtende Membran 8 des Anschlussteils 1 sind derart ausgebildet und angeordnet, dass der in den Innenkonus eingesetzte Kegelschaft 31 der Spritze 32 die geschlitzte Membran 8 zwar öffnet, aber nicht durchdringt (Fig. 6a und 6b).
Damit die aufgespreizte Membran 8 sicher gehalten wird, ist die Membran formschlüssig mit dem unteren Teilstück 2 des Anschlussteils 1 verbunden. Hierzu weist die Membran 8 an der Unterseite eine umlaufende Ausnehmung 37 auf, in die einen umlaufender Vorsprung 38 an der Oberseite des unteren Teilstücks 2 des Anschlussteils 1 greift.

## Patentansprüche

1. Konnektor für medizinische Flüssigkeiten enthaltende Verpackungen, insbesondere Infusions- oder Transfusionsbeutel, mit
einem Anschlussteil (1), der eine kanalförmige Ausnehmung (1 c) aufweist, in der eine geschlitzte selbstabdichtende Membran (8) angeordnet ist, wobei die kanalförmige Ausnehmung eine verpackungsseitige untere und eine anschlussseitige obere Öffnung (1 b, 1a) aufweist,
einem die kanalförmige Ausnehmung verschließenden Abbrechteil (17), der an den Anschlussteil oberhalb der anschlussseitigen Öffnung angeschlossen ist; **dadurch gekennzeichnet,**
**dass** der Anschlussteil (1) oberhalb der selbstabdichtenden Membran (8) als ein Anschlussstück (13) mit einem Innenkonus (14) ausgebildet ist zur abdichtenden Aufnahme eines Kegelschaftes einer Luer-Spritze, wobei das Anschlussstück des Anschlussteils (1) als weiblicher Luer-Konnektor (13) ausgebildet ist und
**dass** der Abbrechteil (17) als flaches Griffstück (19) ausgebildet ist, das mit einer Aussparung (21) versehen ist, die nach Art eines nach unten zeigenden Pfeils ausgebildet ist, der den Konnektor als Zuspritzteil kennzeichnet.

2. Konnektor nach Anspruch 1, **dadurch gekennzeichnet, dass** der Luer-Konnektor (13) des Anschlussteil (1) als weiblicher Luer-Lock-Konnektor (13) mit einem Innenkonus (14) und einem Aussengewinde (15) ausgebildet ist.

3. Konnektor nach einem der Ansprüche 1 bis 2, **dadurch gekennzeichnet, dass** der Abbrechteil (17) über eine Ringbruchzone (16) an den Anschlussteil angeschlossen ist.

4. Konnektor nach einem der Ansprüche 1 bis 3, **dadurch gekennzeichnet, dass** der Anschlussteil (1) aus einem unteren Teilstück (2) und einem oberen Teilstück (3) zusammengesetzt ist, wobei die Teilstücke einschnappend festgelegt sind.

5. Konnektor nach Anspruch 4, **dadurch gekennzeichnet, dass** die selbstabdichtende Membran (8) zwischen dem unteren und oberen Teilstück (3, 4) klemmend gehalten ist.

6. Konnektor nach einem der Ansprüche 1 bis 5, **dadurch gekennzeichnet, dass** die selbstabdichtende Membran (8) einen unteren ringförmigen Abschnitt (9) und einen oberen tellerförmigen Abschnitt (10) aufweist, der eine muldenförmige Vertiefung (11) hat.

7. Konnektor nach Anspruch 6, **dadurch gekennzeichnet, dass** sich an den oberen tellerförmigen Abschnitt (10) ein mittleres Zwischenstück (34) anschliesst, das in den unteren ringförmigen Abschnitt (9) der selbstabdichtenden Membran (8) übergeht.

8. Konnektor nach Anspruch 6 oder 7, **dadurch gekennzeichnet, dass** der ringförmige Abschnitt (9) der selbstabdichtenden Membran (8) zwischen dem unteren und oberen Teilstück (2, 3) des Anschlussteils (1) eingespannt ist.

9. Konnektor nach einem der Ansprüche 6 bis 8, **dadurch gekennzeichnet, dass** der Anschlussteil (1) einen nach innen vorspringenden Ansatz (35) aufweist, an dem sich der ringförmige Abschnitt (9) der selbstabdichtenden Membran (8) abstützt.

10. Konnektor nach einem der Ansprüche 6 bis 9, **dadurch gekennzeichnet, dass** der Anschlussteil (1) einen nach innen vorspringenden Ansatz (36) aufweist, an dem sich der tellerförmige Abschnitt (10) der selbstabdichtenden Membran (8) abstützt.

11. Konnektor nach Anspruch 10, **dadurch gekennzeichnet, dass** der tellerförmige Abschnitt (10) der selbstabdichtenden Membran (8) gegen den nach innen vorspringen Ansatz (36) federnd vorgespannt ist.

12. Konnektor nach einem der Ansprüche 6 bis 8, **dadurch gekennzeichnet, dass** der ringförmige Abschnitt (9) der selbstabdichtenden Membran (8) mit dem unteren Teilstück (2) des Anschlussteils formschlüssig verbunden ist.

13. Konnektor nach einem der Ansprüche 6 bis 12, **dadurch gekennzeichnet, dass** der Innendurchmesser des ringförmigen Abschnitts (9) der selbstabdichtenden Membran (8) kleiner als der Innendurchmesser der kanalförmigen Ausnehmung (1 c) des Anschlussteils (1) ist.

14. Konnektor nach einem der Ansprüche 1 bis 13, **dadurch gekennzeichnet, dass** der Innenkonus (14) des Anschlussstücks (13) und die selbstabdichtende Membran (8) des Anschlussteils (1) derart ausgebildet und angeordnet sind, dass der in den Innenkonus (14) eingesetzte Kegelschaft einer Spritze die geschlitzte Membran zwar öffnet, aber nicht durchdringt.

15. Verpackung für medizinische Flüssigkeiten, insbesondere Infusions- oder Transfusionsbeutel, mit einem Konnektor (20) nach einem der Ansprüche 1 bis 14.

## Claims

1. Connector for packages containing medical liquids, in particular for infusion or transfusion bags, comprising a connection part (1) with a channel-shaped recess (1c) in which is arranged a self-sealing membrane (8) with a slit, the channel-shaped recess having a lower opening (1b) at the package side and an upper opening (1a) at the connection side, and comprising a break-off part (17), which closes the channel-shaped recess and is connected to the connection part above the opening at the connection side,
**characterized in that** the connection part (1) above the self-sealing membrane (8) is designed as a connection piece (13) with an internal cone (14) in order to receive a conical shaft of a Luer syringe in a sealed manner, wherein the connection piece of the connection part (1) is designed as a female Luer connector (13) and **in that** the break-off part (17) is designed as a flat grip (19) which is provided with a cut-out recess (21), which is configured in the manner of a downward-pointing arrow, which identifies the connector as an injection part.

2. Connector according to Claim 1, **characterized in that** the Luer connector (13) of the connection part (1) is designed as a female Luer lock connector (13) with an internal cone (14) and an external thread (15).

3. Connector according to one of Claims 1 to 2, **characterized in that** the break-off part (17) is connected to the connection part via an annular rupture zone (16).

4. Connector according to one of Claims 1 to 3, **characterized in that** the connection part (1) is composed of a lower section (2) and an upper section (3), said sections being secured with a snap fit.

5. Connector according to Claim 4, **characterized in that** the self-sealing membrane (8) is held clamped between the lower and upper sections (2, 3).

6. Connector according to one of Claims 1 to 5, **characterized in that** the self-sealing membrane (8) has a lower annular portion (9) and an upper plate-shaped portion (10), which has a trough-shaped depression (11).

7. Connector according to Claim 6, **characterized in that** the upper plate-shaped portion (10) is adjoined by a central intermediate piece (34), which merges into the lower annular portion (9) of the self-sealing membrane (8).

8. Connector according to Claim 6 or 7, **characterized in that** the annular portion (9) of the self-sealing membrane (8) is clamped between the lower and upper sections (2, 3) of the connection part (1).

9. Connector according to one of Claims 6 to 8, **characterized in that** the connection part (1) has an inwardly projecting shoulder (35), on which the annular portion (9) of the self-sealing membrane (8) rests.

10. Connector according to one of Claims 6 to 9, **characterized in that** the connection part (1) has an inwardly projecting shoulder (36), on which the plate-shaped portion (10) of the self-sealing membrane (8) rests.

11. Connector according to Claim 10, **characterized in that** the plate-shaped portion (10) of the self-sealing membrane (8) is prestressed resiliently against the inwardly projecting shoulder (36).

12. Connector according to one of Claims 6 to 8, **characterized in that** the annular portion (9) of the self-sealing membrane (8) is connected with a form fit to the lower section (2) of the connection part.

13. Connector according to one of Claims 6 to 12, **characterized in that** the internal diameter of the annular portion (9) of the self-sealing membrane (8) is smaller than the internal diameter of the channel-shaped recess (1c) of the connection part (1).

14. Connector according to one of Claims 1 to 13, **characterized in that** the internal cone (14) of the connection piece (13) and the self-sealing membrane (8) of the connection part (1) are designed and arranged in such a way that the conical shaft of a syringe inserted into the internal cone (14) opens the slit membrane but does not pass through it.

15. Package for medical liquids, in particular an infusion or transfusion bag, with a connector (20) according to one of Claims 1 to 14.

## Revendications

1. Connecteur pour conditionnements contenant des liquides médicaux, notamment pour des sachets de perfusion ou de transfusion, comprenant :
une partie de raccordement (1) qui présente un évidement en forme de canal (1c), dans lequel est disposée une membrane (8) autoscellante et fendue, l'évidement en forme de canal présentant une ouverture inférieure du côté du conditionnement et une ouverture supérieure du côté du raccordement (1b, la),
une partie frangible (17) fermant l'évidement en forme de canal, qui est raccordée à la partie de raccordement au-dessus de l'ouverture du côté du raccordement ;
**caractérisé en ce que**
la partie de raccordement (1) est réalisée au-dessus de la membrane (8) autoscellante sous forme de pièce de raccordement (13) avec un cône interne (14) pour recevoir de manière hermétique une tige conique d'une seringue de Luer, la pièce de raccordement de la partie de raccordement (1) étant réalisée sous forme de connecteur de Luer femelle (13) et **en ce que** la partie frangible (17) est réalisée sous forme de pièce de préhension plate (19) qui est pourvue d'un évidement (21) qui est réalisé à la manière d'une flèche orientée vers le bas et qui caractérise le connecteur en tant que partie d'injection.

2. Connecteur selon la revendication 1, **caractérisé en ce que** le connecteur de Luer (13) de la partie de raccordement (1) est réalisé sous forme de connecteur de verrouillage Luer femelle (13) avec un cône interne (14) et un filetage extérieur (15).

3. Connecteur selon l'une quelconque des revendications 1 à 2, **caractérisé en ce que** la partie frangible (17) est raccordée à la partie de raccordement par le biais d'une zone de rupture annulaire (16).

4. Connecteur selon l'une quelconque des revendications 1 à 3, **caractérisé en ce que** la partie de raccordement (1) est constituée d'une pièce partielle inférieure (2) et d'une pièce partielle supérieure (3), les pièces partielles étant fixées par encliquetage.

5. Connecteur selon la revendication 4, **caractérisé en ce que** la membrane (8) autoscellante est maintenue entre la pièce partielle inférieure et la pièce partielle supérieure (3, 4) par serrage.

6. Connecteur selon l'une quelconque des revendications 1 à 5, **caractérisé en ce que** la membrane (8) autoscellante présente une portion inférieure annulaire (9) et une portion supérieure en forme de plateau (10), qui présente un renfoncement en forme de creux (11).

7. Connecteur selon la revendication 6, **caractérisé en ce qu'**une pièce intermédiaire centrale (34) se raccorde à la portion supérieure en forme de plateau (10), et se prolonge dans la portion inférieure annulaire (9) de la membrane (8) autoscellante.

8. Connecteur selon la revendication 6 ou 7, **caractérisé en ce que** la portion annulaire (9) de la membrane (8) autoscellante est serrée entre la pièce partielle inférieure et la pièce partielle supérieure (2, 3) de la partie de raccordement (1).

9. Connecteur selon l'une quelconque des revendications 6 à 8, **caractérisé en ce que** la partie de raccordement (1) présente une projection (35) saillant vers l'intérieur, sur laquelle s'appuie la portion annulaire (9) de la membrane (8) autoscellante.

10. Connecteur selon l'une quelconque des revendications 6 à 9, **caractérisé en ce que** la partie de raccordement (1) présente une projection (36) saillant vers l'intérieur, sur laquelle s'appuie la portion en forme de plateau (10) de la membrane (8) autoscellante.

11. Connecteur selon la revendication 10, **caractérisé en ce que** la portion en forme de plateau (10) de la membrane (8) autoscellante est précontrainte à ressort contre la projection (36) saillant vers l'intérieur.

12. Connecteur selon l'une quelconque des revendications 6 à 8, **caractérisé en ce que** la portion annulaire (9) de la membrane (8) autoscellante est connectée par engagement par correspondance géométrique à la pièce partielle inférieure (2) de la partie de raccordement.

13. Connecteur selon l'une quelconque des revendications 6 à 12, **caractérisé en ce que** le diamètre intérieur de la portion annulaire (9) de la membrane (8) autoscellante est inférieur au diamètre intérieur de l'évidement en forme de canal (1c) de la partie de raccordement (1).

14. Connecteur selon l'une quelconque des revendications 1 à 13, **caractérisé en ce que** le cône interne (14) de la pièce de raccordement (13) et la membrane (8) autoscellante de la partie de raccordement (1) sont réalisés et disposés de telle sorte que la tige conique d'une seringue insérée dans le cône interne (14) ouvre certes la membrane fendue, mais ne la traverse pas.

15. Conditionnement pour liquides médicaux, notamment des sachets de perfusion ou de transfusion, comprenant un connecteur (20) selon l'une quelconque des revendications 1 à 14.
